# EUROPEAN PATENT APPLICATION

(11) **EP 3 943 530 A1**
(43) Date of publication of application: **26.01.2022**
(21) Application number: 19909615.7
(22) Date of filing: 18.03.2019
(51) Int. Cl.: C08J 3/12, A61K 8/73, C08B 3/06, C08L 1/12

(54) **CELLULOSE ACETATE-CONTAINING PARTICLES, COSMETIC COMPOSITION, AND METHOD FOR PRODUCING CELLULOSE ACETATE-CONTAINING PARTICLES**

(71) Applicant: Daicel Corporation, Osaka 530-0001 (JP)
(72) Inventor: KOBAYASHI, Keiko, Minato-ku, Tokyo 1088230 (JP); OMURA, Masaya, Minato-ku, Tokyo 1088230 (JP)
(74) Representative: Grünecker Patent- und Rechtsanwälte PartG mbB
(86) International application number: PCT/JP2019/011161
(87) International publication number: WO 2020/188698

(57) **Abstract**

An object is to provide particles excellent in biodegradability, tactile sensation, and lipophilicity.

Provided are particles containing cellulose acetate, in which the particles have an average particle size of not less than 80 nm and not greater than 100 µm, a sphericity of not less than 0.7 and not greater than 1.0, a degree of surface smoothness of not less than 80% and not greater than 100%, and a surface contact angle with water of not less than 100°; and a total degree of acetyl substitution of the cellulose acetate is not less than 0.7 and not greater than 2.9.

## Description

### Technical Field

The present invention relates to particles containing cellulose acetate, a cosmetic composition, and a method for producing particles containing cellulose acetate.

### Background Art

Various polymer particles which correspond to applications have been proposed in the art. For example, particles are included in cosmetics for various purposes. The purposes for including particles in cosmetics include improving the spread of the cosmetic, changing the tactile sensation, imparting a wrinkle blurring effect, and improving the lubricity of a foundation or the like.

In particular, particles with high sphericity is excellent in imparting tactile sensation and provides a light-scattering (soft-focus) effect depending on the physical properties and shape of the particles. Such particles when used in a foundation or the like fill and smooth the roughness of the skin, scatter the light in various directions, and thus can be expected to have an effect of making wrinkles and the like less noticeable (soft-focus effect).

For such a purpose and an effect of cosmetics, particles included in cosmetics are required to have a narrow particle size distribution and high sphericity. Examples of such particles include particles made of a synthetic polymer, such as polyamide (such as nylon 12), polymethyl methacrylate (PMMA), and polystyrene (PS), which have been proposed in the art.

However, particles made of these synthetic polymers are light-weight having a relative density of not greater than 1 and have too small particle size, thus easily float on water. Therefore, the particles may not be removed at a sewage treatment facility, and may flow as they are into the river and further into the sea through the river. This causes a problem of the particles made of these synthetic polymers polluting the ocean and the like.

Furthermore, particles made of these synthetic polymers have the characteristics of adsorbing trace amounts of chemical pollutants in the environment. Therefore, there are concerns that the particles may cause various effects; for example, planktons and fish swallow particles that have adsorbed the chemical pollutants, and this can also negatively affect the humans.

From such concerns, attempts have been made to replace particles of a synthetic polymer used in various applications with biodegradable particles. In particular, there is also a proposal to replace the synthetic polymer with cellulose, a natural ingredient.

In addition, examples of representative biodegradable polymer include cellulose acetate. Cellulose acetate is advantageously obtained from natural materials, such as wood and cotton, without conflicting with food or feed resources. Thus, it is beneficial if particles of a synthetic polymer are replaced with particles of cellulose acetate. However, in a method for producing particles of a synthetic polymer, the applicable polymer is limited, and applying the method to the production of particles of cellulose acetate is difficult.

Patent Document 1 describes a method including: forming a polysaccharide ester product from a polysaccharide synthesis, in which the polysaccharide ester product contains a polysaccharide ester and a solvent; diluting the polysaccharide ester product and thereby yielding a polysaccharide ester dope; and forming a plurality of polysaccharide ester microspheres from the polysaccharide ester dope; and lists a cosmetic composition as an article that can contain a polysaccharide ester microsphere.

Patent Document 2 describes a cellulose acylate having a volume average particle size D50 of not less than 72 µm and not greater than 100 µm, as measured using a laser diffraction particle size distribution measuring apparatus, a degree of polymerization of not less than 131 and not greater than 350, and a degree of substitution of not less than 2.1 and not greater than 2.6; and also describes that a method for producing the cellulose acylate is preferably a method for producing a cellulose acylate, the method including: acylating cellulose in the presence of sulfuric acid; and deacylating the acylated cellulose in a polar solvent in the presence of acetic acid.

Patent Document 3 describes producing a molded article, including kneading a resin component (A), such as a thermoplastic resin, and a water-soluble auxiliary component (B) to prepare a dispersion; and eluting the auxiliary component (B) from the dispersion to produce a molded article (e.g., a porous article or spherical particles) constituted of the resin component (A); and also describes a cellulose derivative, such as cellulose acetate, as the resin component (A).

In addition, surface treatment of particles has been also proposed in the art. For example, Patent Document 4 describes that, to provide a powder cosmetic forming a cosmetic film having good water resistance and the like, a powder is subjected to surface treatment to form a powder in which not less than 90 wt.% of a total powder amount exhibits hydrophobicity.

Patent Document 4 specifically describes the following. Examples of the hydrophobizing treatment of a powder commonly known in the art include a method of reacting a metal hydroxide and a higher fatty acid on a powder surface; a method of coating a powder surface with a silicone resin and baking; a method of coating a powder surface with dimethylpolysiloxane or methylhydrogenpolysiloxane, heating, or baking, as necessary, using a crosslinking polymerization catalyst; a method of coating a powder surface with alkylpolysiloxane and baking; a method of uniformly mixing a powder and a metal hydroxide or an acidic material, and performing crosslinking-polymerization of methylhydrogenpolysiloxane on a powder surface; a method of mechanochemically treating a powder with a high molecular weight silicone; a method of polymerizing a cyclic organosiloxane on a powder surface in a vapor phase at a relatively low temperature; and a method of treating a powder surface in an aqueous solution of an organic fluorine-based compound, such as a perfluoroalkyl group-containing phosphoric acid derivative. This powder is exemplified by inorganic powders, such as that of titanium oxide or zirconium oxide; resins, such as that of nylon or a silicone elastomer; and organic powders, such as that of cellulose or silk.

Patent Document 5 proposes a microcrystalline cellulose powder surface-treated with metal soap or hydrogenated lecithin and describes surface-treating a white fine powder cellulose microcrystalline aggregate with metal soap or hydrogenated lecithin, the white fine powder cellulose microcrystalline aggregate obtained by acid hydrolysis or alkaline hydrolysis of a natural cellulose material, such as cotton, linter, or pulp, and having an average degree of polymerization in a range of 75 to 375. In addition, Patent Document 5 describes that the ratio (L/D) of the major axis length (L) and the minor axis length (D) of the particles is preferably not greater than L/D 3.

### Citation List

### Patent Document

Patent Document 1: JP 2016-500129 A
Patent Document 2: JP 6187653 B
Patent Document 3: JP 2004-051942 A
Patent Document 4: JP 11-222411 A
Patent Document 5: JP 2003-146829 A

### Summary of Invention

### Technical Problem

However, the polysaccharide ester microspheres of Patent Document 1 are porous particles having a large particle size and a broad particle size distribution and thus are not sufficient as an alternative to particles of a synthetic polymer to be contained in cosmetics or the like. The cellulose acylate obtained by the production method described in Patent Document 2 is also porous particles of an undefined form. In addition, the particulate molded article obtained by the production method described in Patent Document 3 also has a low sphericity and is particles that are approximately spherical. Even if a cellulose is subjected to a surface treatment, such as that in Patent Document 4, milled cellulose tends to be of an undefined shape and not a sphere. Furthermore, the particles disclosed in Patent Document 5 have a shape far from the spherical particles as can be seen from the value of the ratio of the major axis length (L) and the minor axis length (D) of the particles.

Particles made of cellulose, cellulose acetate, and the like known in the art have excellent biodegradability but are not spherical particles, and even the particles that have been surface-modified and lipophilized (in other words, hydrophobized) fail to provide cosmetics having excellent tactile sensation.

Furthermore, for cosmetics, in particular, make-up cosmetics, an oily component is used in a large amount to improve water resistance and improve make-up sustainability. However, biodegradable particles known in the art have poor lipophilicity and thus have poor dispersibility in an oily component.

An object of the present invention is to provide particles excellent in biodegradability, tactile sensation, and lipophilicity.

### Solution to Problem

A first aspect of the present invention relates to particles containing cellulose acetate, in which the particles have an average particle size of not less than 80 nm and not greater than 100 µm, a sphericity of not less than 0.7 and not greater than 1.0, a degree of surface smoothness of not less than 80% and not greater than 100%, and a surface contact angle with water of not less than 100°; and a total degree of acetyl substitution of the cellulose acetate is not less than 0.7 and not greater than 2.9.

In the particles, the surface contact angle with water may be not less than 120°.

In the particles, the total degree of acetyl substitution of the cellulose acetate may be not less than 2.0 and less than 2.6.

In the particles, the particles may contain a plasticizer, and a content of the plasticizer may be not greater than 1 wt.% relative to a weight of the particles.

In the particles, the plasticizer may be at least one or more selected from the group consisting of a citrate-based plasticizer, a glycerin ester-based plasticizer, an adipate-based plasticizer, and a phthalate-based plasticizer.

In the particles, the glycerin ester-based plasticizer may be triacetin.

A second aspect of the present invention relates to a cosmetic composition containing particles.

The third aspect of the present invention relates to a method for producing the particles, the method including surface-treating cellulose acetate particles with a lipophilicity-imparting agent, in which the cellulose acetate particles have an average particle size of not less than 80 nm and not greater than 100 µm, a sphericity of not less than 0.7 and not greater than 1.0, and a degree of surface smoothness of not less than 80% and not greater than 100%; and a total degree of acetyl substitution of the cellulose acetate is not less than 0.7 and not greater than 2.9.

In the method for producing the particles, the lipophilicity-imparting agent may contain a silicone-based component.

In the method for producing the particles, the surface treatment may be a surface treatment by a wet treatment method.

### Advantageous Effects of Invention

An embodiment of the present invention can provide particles excellent in biodegradability, tactile sensation, and lipophilicity.

### Brief Description of Drawing

FIG. 1 is an image illustrating a method for evaluating the degree of surface smoothness (%).
FIG. 2 is an image illustrating a method for evaluating the degree of surface smoothness (%).
FIG. 3 is a photograph of a water droplet in measuring a contact angle (Example 2).
FIG. 4 is a photograph of a water droplet in measuring a contact angle

### (Comparative Example 2)

### Description of Embodiments

### Particles containing cellulose acetate

Particles containing cellulose acetate of the present disclosure are particles containing cellulose acetate, in which the particles have an average particle size of not less than 80 nm and not greater than 100 µm, a sphericity of not less than 0.7 and not greater than 1.0, a degree of surface smoothness of not less than 80% and not greater than 100%, and a surface contact angle with water of not less than 100°; and a total degree of acetyl substitution of the cellulose acetate is not less than 0.7 and not greater than 2.9.

The average particle size of the particles containing cellulose acetate of the present disclosure is not less than 80 nm and not greater than 100 µm and may be not less than 100 nm, not less than 1 µm, not less than 2 µm, or not less than 4 µm. In addition, the average particle size may be not greater than 80 µm, not greater than 40 µm, not greater than 20 µm, not greater than 14 µm, or not greater than 10 µm. The particles with too large average particle size may have poor tactile sensation and a reduced light-scattering (soft-focus) effect. Alternatively, the particles with too small average particle size may be difficult to produce. Here, the tactile sensation includes, for example, skin feel and tactile sensation of a cosmetic composition containing the particles, in addition to tactile sensation in directly touching the particles containing cellulose acetate.

The average particle size can be measured using dynamic light scattering, specifically as follows. First, the particles are suspended at a concentration of 100 ppm in pure water using an ultrasonic vibrating apparatus to prepare a sample. Then, the average particle size can be measured by measuring the particle size volume distribution by laser diffraction ("Laser Diffraction/Scattering Particle Size Distribution Measuring Apparatus LA-960" available from Horiba Ltd., ultrasonic treatment for 15 minutes, and a refractive index (1.500, medium (water; 1.333)). The average particle size herein refers to the value of the particle size corresponding to 50% of the integrated scattering intensity in this particle size distribution.

The coefficient of variation of the particle size of the particles containing cellulose acetate of the present disclosure may be not less than 0% and not greater than 60%, or not less than 2% and not greater than 50%.

The coefficient of variation (%) of the particle size can be calculated by an equation: (standard deviation of particle size)/(average particle size) x 100.

The sphericity of the particles containing cellulose acetate of the present disclosure is not less than 0.7 and not greater than 1.0, preferably not less than 0.8 and not greater than 1.0, more preferably not less than 0.9 and not greater than 1.0. The particles with a sphericity of less than 0.7 have poor tactile sensation, and, for example, a cosmetic composition containing such particles have a reduced soft-focus effect.

The sphericity can be measured by the following method. Using an image of particles observed with a scanning electron microscope (SEM), the major axis length and the minor axis length of 30 randomly selected particles are measured, and the (minor axis length)/(major axis length) ratio of each particle is determined. Then, the average value of the (minor axis length)/(major axis length) ratios is defined as the sphericity. The closer to 1 the sphericity is, the closer to the true sphere the particles can be determined to be.

The degree of surface smoothness of the particles containing cellulose acetate of the present disclosure is not less than 80% and not greater than 100%, preferably not less than 85% and not greater than 100%, more preferably not less than 90% and not greater than 100%. The particles with the degree of surface smoothness of less than 80% may have poor tactile sensation. The degree of surface smoothness is preferably closer to 100% in terms of tactile sensation.

The degree of surface smoothness can be determined as follows: a scanning electron micrograph of the particles is obtained, recesses and protrusions of the particle surfaces are observed, and the degree of surface smoothness is determined based on the area of recessed portions on the surfaces.

The surface contact angle of the particles containing cellulose acetate of the present disclosure with water is not less than 100°, preferably not less than 120°, and more preferably not less than 130°. In terms of compatibility with a cosmetic general-purpose oil agent and hydrophobicity, the surface contact angle may be not greater than 180°. The particles with a surface contact angle of less than 100° are considered to have insufficient lipophilicity.

The surface contact angle with water can be determined as follows: a planer surface is formed with the particles (powder), a water droplet dropped on the planer surface is observed, and the surface contact angle is determined using the θ/2 method. Specifically, a fully automatic contact angle meter (analysis software: interFAce Measurement and Analysis System FAMAS, available from Kyowa Interface Science Co., Ltd.) can be used as the measurement apparatus.

The total degree of acetyl substitution of the cellulose acetate of the particles containing cellulose acetate of the present disclosure is not less than 0.7 and not greater than 2.9, preferably not less than 0.7 and not greater than 2.6, more preferably not less than 1.0 and not greater than 2.6, even more preferably not less than 1.4 and not greater than 2.6, and most preferably not less than 2.0 and not greater than 2.6.

The cellulose acetate with a total degree of acetyl substitution of less than 0.7 increases water solubility and may elute in step of producing cellulose acetate particles in production of the particles containing cellulose acetate described below, particularly in removing a water-soluble polymer from a dispersion. This may reduce the sphericity of the resulting particles and thus result in poor tactile sensation of the resulting particles containing the cellulose acetate. On the other hand, the cellulose acetate with a total degree of acetyl substitution of greater than 2.9 may result in poor biodegradability of the particles containing the cellulose acetate.

The total degree of acetyl substitution of the cellulose acetate can be measured by the following method. First, the total degree of acetyl substitution is the sum of each degree of substitution at the 2-, 3-, and 6-positions of the glucose ring of the cellulose acetate, and each degree of acetyl substitution at the 2-, 3-, and 6-positions of the glucose ring of the cellulose acetate can be measured by NMR according to the method of Tezuka (Tezuka, Carbonydr. Res. 273, 83 (1995)). That is, the free hydroxyl group of a cellulose acetate sample is propionylated with propionic anhydride in pyridine. The resulting sample is dissolved in deuteriochloroform, and the ¹³C-NMR spectrum is measured. The carbon signals of the acetyl group appear in the region from 169 ppm to 171 ppm in the order of the 2-, 3-, and 6-positions from the high magnetic field; and the carbonyl carbon signals of the propionyl group appear in the region from 172 ppm to 174 ppm in the same order. Each degree of acetyl substitution at the 2-, 3-, and 6-positions of the glucose ring in the original cellulose acetate can be determined from the presence ratio of the acetyl group and the propionyl group at the respective corresponding positions. The degree of acetyl substitution can be analyzed by ¹H-NMR in addition to ¹³C-NMR

Furthermore, the total degree of acetyl substitution is determined by converting the degree of acetylation determined according to the method for measuring the degree of acetylation in ASTM: D-817-91 (Testing methods for cellulose acetate, etc.). This is the most common procedure to determine the degree of substitution of cellulose acetate.
DS = 162.14 x AV x 0.01/(60.052 - 42.037 x AV x 0.01)

In the above equation, DS is the total degree of acetyl substitution, and AV is the degree of acetylation (%). Note that the value of the degree of substitution obtained by the conversion usually has a slight discrepancy from the value measured by NMR described above. When the converted value and the value measured by NMR are different, the value measured by NMR is adopted. In addition, if the value varies among the specific methods of NMR measurement, the value measured by NMR according to the method of Tezuka described above is adopted.

The method for measuring the degree of acetylation according to ASTM: D-817-91 (Testing methods for cellulose acetate, etc.) is outlined as follows. First, 1.9 g of dried cellulose acetate is accurately weighed and dissolved in 150 mL of a mixed solution of acetone and dimethyl sulfoxide (a volume ratio of 4:1), then 30 mL of a 1 N sodium hydroxide solution is added, and the cellulose acetate is saponified at 25°C for 2 hours. Phenolphthalein is added as an indicator, and excess sodium hydroxide is titrated with a 1 N sulfuric acid (concentration factor: F). In addition, a blank test is performed in the same manner as described above, and the degree of acetylation is calculated according to the following equation.
Average degree of acetylation (%) = {6.5 x (B-A) x F}/W
where A represents the titration volume (mL) of the 1 N sulfuric acid for the sample, B represents the titration volume (mL) of the 1 N sulfuric acid for the blank test, F represents the concentration factor of the 1 N sulfuric acid, and W represents the weight of the sample.

The particles containing cellulose acetate of the present disclosure may have a bulk density of not less than 0.1 and not greater than 0.9, or not less than 0.5 and not greater than 0.9. For example, for a cosmetic containing the particles, the higher the bulk density of the particles, the better the flowability of the cosmetic composition is. The bulk density can be measured by a method in accordance with JIS K 1201-1.

### Optional component

The particles containing cellulose acetate of the present disclosure may or may not contain a plasticizer. In the present disclosure, the plasticizer refers to a compound capable of increasing the plasticity of the cellulose acetate. The plasticizer is not particularly limited, and examples include adipate-based plasticizers containing an adipate ester, such as dimethyl adipate, dibutyl adipate, diisostearyl adipate, diisodecyl adipate, diisononyl adipate, diisobutyl adipate, diisopropyl adipate, diethylhexyl, adipate dioctyl adipate, dioctyldodecyl adipate, dicapryl adipate, and dihexyldecyl adipate; citrate-based plasticizers containing a citrate ester, such as acetyl triethyl citrate, acetyl tributyl citrate, isodecyl citrate, isopropyl citrate, triethyl citrate, triethylhexyl citrate, and tributyl citrate; glutarate-based plasticizers containing a glutarate ester, such as diisobutyl glutarate, dioctyl glutarate, and dimethyl glutarate; succinate-based plasticizers containing a succinate ester, such as diisobutyl succinate, diethyl succinate, diethylhexyl succinate, and dioctyl succinate; sebacate-based plasticizers containing a sebacate ester, such as diisoamyl sebacate, diisooctyl sebacate, diisopropyl sebacate, diethyl sebacate, diethylhexyl sebacate, and dioctyl sebacate; glycerin ester-based plasticizers containing a glycerin alkyl ester, such as triacetin, diacetin, and monoacetin; neopentyl glycol; phthalate-based plasticizers containing a phthalate ester, such as ethyl phthalate, methyl phthalate, diaryl phthalate, diethyl phthalate, diethylhexyl phthalate, dioctyl phthalate, dibutyl phthalate, and dimethyl phthalate; and phosphate-based plasticizers containing a phosphate ester, such as trioleil phosphate, tristearyl phosphate, and tricetyl phosphate. In addition, examples also include di-2-methoxyethyl phthalate dibutyl tartrate ethyl o-benzoyl benzoate, ethyl phthalyl ethyl glycolate (EPEG), methyl phthalyl ethyl glycolate (MPEG), N-ethyl toluene sulfonamide, p-toluenesulfonate o-cresyl triethyl phosphate (TEP), triphenyl phosphate (TPP), and tripropionin. These plasticizers may be used alone, or two or more of the plasticizers may be used in combination.

Among them, the plasticizer is preferably at least one or more selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; adipate-based plasticizers, such as diisononyl adipate; and phthalate-based plasticizers, such as ethyl phthalate and methyl phthalate; more preferably at least one or more selected from the group consisting of triethyl citrate, acetyl triethyl citrate, acetyl tributyl citrate, triacetin, and diisononyl adipate; even more preferably at least one or more selected from the group consisting of acetyl triethyl citrate, triacetin, diacetin, and diethyl phthalate; and most preferably triacetin. Note that a phthalate-based plasticizer must be used with care because of concerns about similarity to environmental hormones.

When the particles containing cellulose acetate contains a plasticizer, the content of the plasticizer included in the particles containing cellulose acetate is not particularly limited. For example, the content may be greater than 0 wt.% relative to the weight of the particles containing cellulose acetate. In addition, the content may be not greater than 40 wt.%, not greater than 30 wt.%, not greater than 20 wt.%, not greater than 10 wt.%, not greater than 5 wt.%, or not greater than 1 wt.%.

The content of the plasticizer in the particles containing cellulose acetate is determined by ¹H-NMR measurement.

The particles containing cellulose acetate of the present disclosure may contain a lipophilicity-imparting agent. In the present disclosure, the lipophilicity-imparting agent refers to a compound capable of being deposited on the cellulose acetate particles and increasing the lipophilicity of the cellulose acetate particles. Here, "being deposited on" the cellulose acetate particles includes both being deposited on or being supported on at least a portion of the surfaces of the cellulose acetate particles, and covering and being deposited on the entire surfaces of the cellulose acetate particles. Covering the entire surfaces of the cellulose acetate particles can also be referred to as coating.

The lipophilicity-imparting agent preferably coats the surfaces of the cellulose acetate particles.

The lipophilicity-imparting agent is not particularly limited, and examples include a lipid component (the lipid component includes a lecithin component), a silicone-based component, a metal soap-based component, a fluorine-based component, an amino acid-based component, and a ceramide-based component. One lipophilicity-imparting agent may be contained alone, or two or more in combination.

Among them, the lipophilicity-imparting agent preferably contains a glycerophospholipid among the lipid components described later and, in particular, preferably contains lecithin among the glycerophospholipids. This is because glycerophospholipids, especially lecithin, are the major constituents of biomembranes and are highly safe.

In addition, the lipophilicity-imparting agent preferably contains a silicone-based component. The silicone-based component has properties of being physiologically inert, highly safe, and stable, and thus is suitable for using the particles containing cellulose acetate particularly in cosmetics directly touching the skin.

Examples of the lipid components include fats, fatty acid esters, waxes, higher alcohols, phospholipids, and other lipid components. In addition, the lipid components also include components separated from these lipid components and cured products obtained by hydrogenation of these lipid components.

Examples of the fats include solid fats, such as cocoa butter, coconut oil, horse oil, hydrogenated coconut oil, palm oil, beef tallow, mutton tallow, and hydrogenated castor oil.

Examples of the waxes include hydrocarbons, such as polyethylene wax, paraffin wax (a linear hydrocarbon), microcrystalline wax (a branched saturated hydrocarbon), ceresin wax, Japanese wax, montan wax, and Fischer-Tropsch wax; beeswax, lanolin, carnauba wax, candelilla wax, rice bran wax (rice wax), spermaceti wax, jojoba oil, bran wax, montan wax, kapok wax, bayberry wax, shellac wax, sugarcane wax, isopropyl lanolin fatty acid, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether.

Examples of the higher alcohols include higher fatty acids, such as myristic acid, palmitic acid, stearic acid, and behenic acid; cetyl alcohol, stearyl alcohol, behenyl alcohol, myristyl alcohol, and cetostearyl alcohol.

Examples of the phospholipids include glycerophospholipid. Lecithin among the glycerophospholipids refers to lipid products containing a phospholipid, and examples of a lecithin-based component include soy lecithin, egg yolk lecithin, and hydrogenated lecithin.

Examples of the other lipid components include trimethylsiloxysilicate, dimethylamino methacrylate quaternized salts, vinylpyrrolidone-methacrylate-N,N-dimethyl-ethylantinioethyl salt copolymers, silicone/polyether-based polyurethane resins, (methacryloyloxyethyl carboxybetaine/methacrylalkyl) copolymers, dextrin, (vinylpyrrolidone/VA) copolymers, ammonium alkyl acrylate copolymers, polyvinyl alcohol, ethyl polyacrylate, (alkyl acrylate/octyl acrylamide) copolymers, (acrylates/propyl trimethicone methacrylate) copolymers, polyvinyl acetate, (acrylates/dimethicone) copolymers, and 3-[tris(trimethylsiloxane)silyl]-propyl carbamate pullulan.

The silicone-based component refers to components containing a structure represented by Formula (1) or (2) below. Rs in Formulas (1) and (2) below each represent an alkyl group.

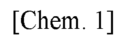

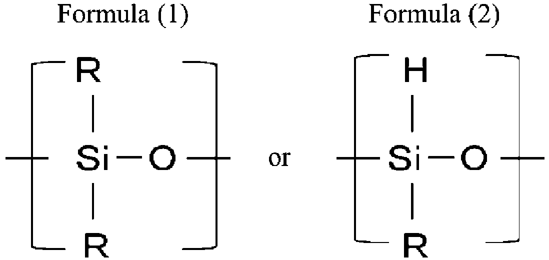

Examples of the silicone-based component include chain silicone oils, such as methylhydrogenpolysiloxane (methicone), polydimethylsiloxane (dimethicone), hydrogen dimethicone ((dimethicone/methicone) copolymer), dimethylsiloxane-methylphenylsiloxane copolymer, and methylphenylpolysiloxane; cyclic dimethylsiloxanes, such as octamethylcyclotetrasiloxane, decamethylcyclopentasiloxane, and dodecamethylcyclohexasiloxane; cyclic methylhydrogenpolysiloxanes; dimethiconols; silicone-based components having two or more different reactive groups in a molecule, such as trimethoxycaprylylsilane, triethoxycaprylylsilane, and aminopropyltriethoxysilane; graft polymers, such as (alkyl acrylate/dimethicone) copolymers, and polyether graft acrylic silicone; modified silicone resins, such as fluoro-modified silicone resins; and modified silicone oils, such as epoxy-modified silicone oils, carboxyl-modified silicone oils, methacrylic-modified silicone oils, alcohol-modified silicone oils, mercapto-modified silicone oils, vinyl-modified silicone oils, amino-modified silicone oils, polyether-modified silicone oils, higher fatty acid-modified silicone oils, and terminal-reactive silicone oils.

Examples of the methylhydrogenpolysiloxane (methicone) include those of a structural formula represented by Formula (3) below (where k is an average number and k = 7 to 30).

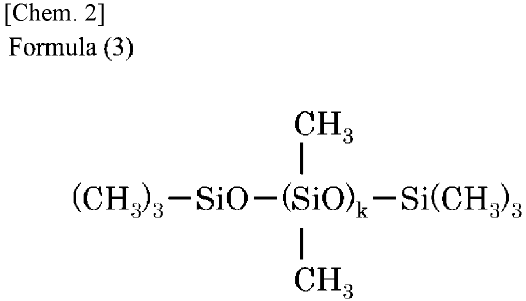

Examples of the cyclic methylhydrogenpolysiloxane include those having a general chemical formula represented by Formula (4) below.

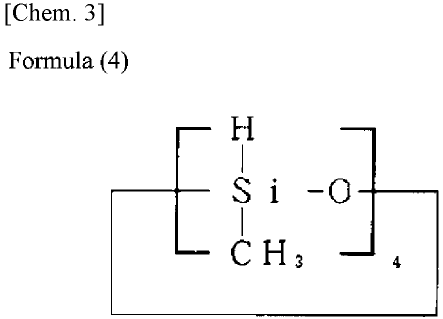

Among the silicone-based components, polydimethylsiloxane (dimethicone), a dimethylsiloxane-methylphenylsiloxane copolymer, and cyclic dimethyl siloxane are preferred.

The metal soap-based component is a metal salt of a long-chain fatty acid other than sodium and potassium salts of a long-chain fatty acid. Examples of the metal soap-based component specifically include aluminum stearate, magnesium stearate, aluminum myristate, aluminum dimyristate, and isopropyl titanium triisostearate.

The metal soap-based component has properties of being insoluble or almost insoluble in water but excellent in solubility in oil. The metal soap-based component forms hydrogen bonds with unsubstituted hydroxyl groups of cellulose acetate by the action of the metal. Thus, the metal soap-based component has an advantage of being capable of surface-treating the cellulose acetate particles only by being supported on the surfaces of the particles. That is, this eliminates the need for curing. A cosmetic composition containing cellulose acetate particles imparted with lipophilicity prepared using a metal soap-based component as a lipophilicity-imparting agent can have increased viscosity. This is particularly the case for an oil-rich liquid type cosmetic composition.

The fluorine-based component refers to a component having a C-F bond (carbon-fluorine bond).

Examples of the fluorine-based component include phosphoric acid derivatives having a perfluoroalkyl group, such as perfluoroalkyl phosphate esters, amine salts of perfluoroalkyl phosphate esters, those represented by Formula (5) below, and those represented by Formula (6) below; and phosphoric acid derivatives having a perfluoropolyether group. In addition, examples include perfluoroalkylsilanes, such as perfluorooctyltriethoxysilane and those represented by Formula (7) below.

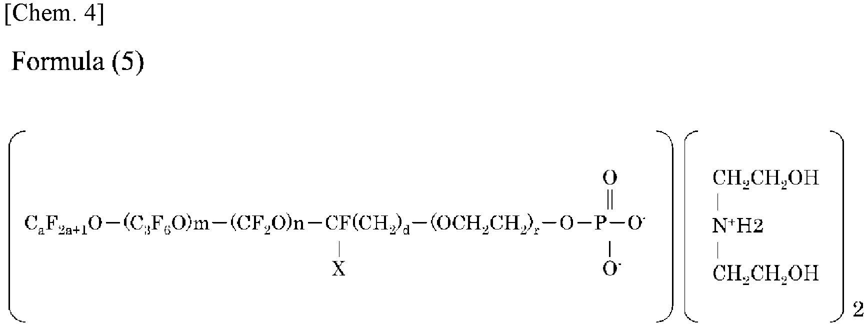

In Formula (5) above, m/n is from 1 to 100 and more preferably from 20 to 40; a represents from 1 to 10; d represents from 0 to 2; r represents from 1 to 2; and X represents F or CF₃. In Formula (5), the molecular weight of the perfluoropolyether group is preferably not less than 300 and more preferably not less than 500.

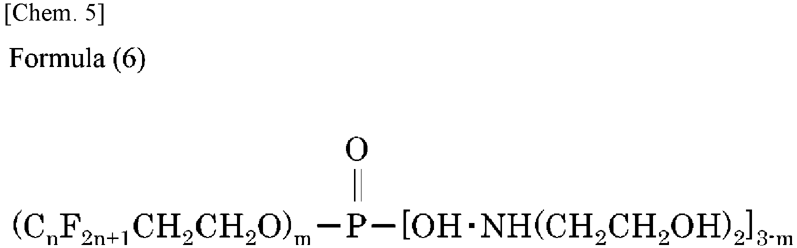

In Formula (6) above, n represents an integer of 6 to 18; and m represents 1 or 2.

Formula (7): CₐF2ₐ₊₁(CH₂)_{b}SiX₃

In Formula (7) above, a represents an integer of 1 to 12; b represents an integer from 1 to 5; and X is identical or different and represents an alkoxy group, a halogen atom, or an alkyl group; however, excluding a case where all Xs are alkyl groups.

Examples of the amine salts of perfluoroalkyl phosphate esters include commercially available products, such as Asahi Guard AG530 (available from Asahi Glass Co., Ltd.). Examples of the perfluoroalkylsilanes include LS-160, LS-360, LS-912, LS-1080, LS-1090, and LS-1465 (these are available from Shin-Etsu Chemical Co., Ltd.), and XC95-418, XC95-466, XC95-467, XC95-468, XC95-469, XC95-470, XC95-471, and XC95-472 (these are available from Toshiba Silicone Co., Ltd.).

The amino acid-based component refers to a component having a structure represented by Formula (8) below.

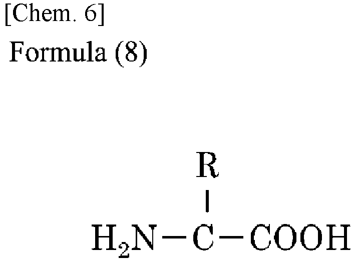

In Formula (8) above, a saturated or unsaturated aliphatic ester group having from 8 to 40 carbon atoms represented by -CR-COOH is bonded onto the nitrogen atom of an amino acid represented by the chemical structural formula.

Examples of the amino acid-based component include disodium N-stearoyl-L-glutamate, sodium lauroyl glutamate, sodium lauroyl aspartate, magnesium palmitoyl glutamate, lauroyl lysine, and octanoyl lysine.

Ceramide is a type of sphingolipid and refers to a compound in which sphingosine and a fatty acid are amide-bonded. Examples of the ceramide-based component include "bioceramides" (human ceramides), animal-derived "natural ceramides" (animal ceramides), plant-derived "plant ceramides", and chemically synthesized "pseudoceramides" (synthetic ceramides). Pseudoceramides may be referred to as synthetic ceramides or synthetic pseudoceramides.

The ceramide that human skin naturally has is composed of seven types of "ceramide 1" to "ceramide 7". "Ceramide 2" has a high moisture retention function and accounts for approximately 20% of the total ceramide. The synthetic pseudo-ceramides have a structure close to this "ceramide 2" and thus is preferred. Examples of the synthetic pseudo-ceramide include hydroxypropyl bis partamide MEA and hexadecyloxy PG hydroxyethyl hexadecanamide.

The sphingosines constituting ceramides may be various amide derivatives having a structure similar to that of natural sphingosine derivatives, that is, synthetic sphingosine analogs. Examples of the synthetic sphingosine analog include a ceramide analog of Formula (9) below.

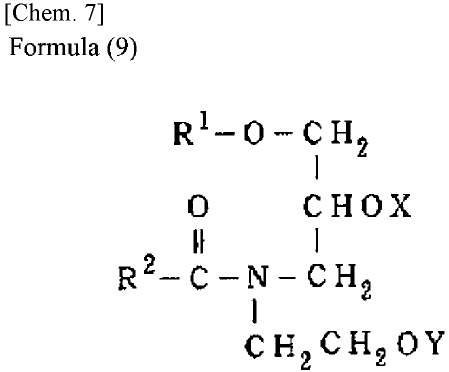

In Formula (9) above, R¹ represents a linear or branched, saturated or unsaturated alkyl group having from 10 to 26 carbon atoms; R² represents a linear or branched, saturated or unsaturated alkyl group having from 9 to 25 carbon atoms; and X and Y each represent a hydrogen atom or a saccharide residue.

When the particles containing cellulose acetate contains a lipophilicity-imparting agent, the content of the lipophilicity-imparting agent contained in the particles containing cellulose acetate is not particularly limited, but if the lipophilicity-imparting agent is localized on the surface, the content may be not less than 0.005 wt.%, preferably not less than 0.01 wt.% and not greater than 50 wt.%, more preferably not less than 1 wt.% and not greater than 10 wt.%, and even more preferably not less than 1.5 wt.% and not greater than 5 wt.% relative to the weight of the particles containing cellulose acetate.

With a content of the lipophilicity-imparting agent of less than 0.005 wt.%, the lipophilicity may be too low, and with a content of the lipophilicity-imparting agent of greater than 50 wt.%, the particles may readily aggregate or fix.

Methods for measuring the content of the lipophilicity-imparting agent in the particles containing cellulose acetate are described. A lipophilicity-imparting agent containing an element, such as silicon, not constituting cellulose acetate can be determined by elemental analysis. In addition, a lipophilicity-imparting agent present on the surface is extracted with a suitable extraction solvent and can be analyzed with a high-speed liquid chromatograph or the like. In particular, the lecithin-based component is preferably analyzed with a high-speed liquid chromatograph or the like. These analyses are described in Mutsuhito WATANABE, Toshihiro ITO, Masaaki IIDA, Atsuyoshi OKABE, Yoshinari KAWAGUCHI, Kikuo SHINBO, Ryoji SONO, Tateo MURUI, and Toshiyuki KANEKO, Yukagaku (Journal of Japan Oil Chemists' Society), 35, 1018 (1986).

The particles containing cellulose acetate of the present disclosure have excellent biodegradability. The biodegradation rate is preferably not less than 40 wt.% and more preferably not less than 50 wt.% within 30 days and may be not greater than 80 wt.% or not greater than 60 wt.% or less.

The biodegradation rate can be measured by a method using activated sludge in accordance with JIS K6950.

The particles containing cellulose acetate of the present disclosure can be produced by a production method described later.

The particles containing cellulose acetate of the present disclosure is excellent in biodegradability, tactile sensation, and lipophilicity, and thus can be suitably used, for example, in cosmetic compositions. In addition, the particles containing cellulose acetate have high sphericity, and thus the particles containing cellulose acetate, when included in a cosmetic composition, fill in and smooth out the roughness of the skin and scatter the light in various directions, thus providing an effect of making wrinkles and the like less noticeable (soft-focus effect). The lipophilicity of the particles is important particularly in preparing make-up cosmetics. The make-up cosmetics are formed by a mixed dispersion of particles and an oil agent. The important point here is good compatibility (dispersibility) between the particles and the oil agent. Even for the particles made of cellulose acetate, improvement of the dispersibility of the particles in oil when used in make-up cosmetics can increase the stability of the cosmetic compositions.

Examples of the cosmetic composition include foundations, such as liquid foundations and powder foundations; concealers; sunscreens; makeup bases; lipsticks and lipstick bases; facial powders, such as body powders, solid white powders, and face powders; solid powder eye shadows; wrinkle masking creams; and skin and hair external preparations mainly for cosmetic purposes, such as skin care lotions; and the dosage form is not limited. The dosage form may be any of a liquid preparation, such as an aqueous solution, a milky lotion, and a suspension; a semi-solid preparation, such as a gel and a cream; or a solid preparation, such as a powder, a granule, and a solid. In addition, the dosage form may be an emulsion preparation, such as a cream and a milky lotion; an oil gel preparation, such as a lipstick; a powder preparation, such as a foundation; an aerosol preparation, such as a hair styling agent; or the like.

### Method for producing particles containing cellulose acetate

A method for producing particles containing cellulose acetate of the present disclosure includes surface-treating cellulose acetate particles with a lipophilicity-imparting agent. In addition, the particles containing cellulose acetate have an average particle size of not less than 80 nm and not greater than 100 µm, a sphericity of not less than 0.7 and not greater than 1.0, a degree of surface smoothness of not less than 80% and not greater than 100%, and a surface contact angle with water of not less than 100°; and a total degree of acetyl substitution of the cellulose acetate is not less than 0.7 and not greater than 2.9.

### Surface treatment

Surface treatment of the cellulose acetate particles with a lipophilicity-imparting agent can provide particles having excellent lipophilicity and excellent dispersibility in an oily component. The method of the surface treatment is not particularly limited as long as the method can deposit the lipophilicity-imparting agent on the surfaces of the cellulose acetate particles. In addition, the method of the surface treatment may be those in which the lipophilicity-imparting agent deposited on the cellulose acetate particles forms an ionic bond, crosslinks, or polymerizes with the cellulose acetate particles.

Examples of depositing the lipophilicity-imparting agents on the surfaces of the cellulose acetate particles include a method of simply utilizing physico-chemical bonding. Specifically, examples include a method of dissolving a lipophilicity-imparting agent in a suitable solvent, adding cellulose acetate particles to the solution to deposit the lipophilicity-imparting agent on the surfaces of the cellulose acetate particles; a method of utilizing an electric charge on the surfaces of the cellulose acetate particles, ionically bonding and depositing a compound having a high polar group or an ionic group on the surfaces of the cellulose acetate particles; and a method of utilizing a functional group on the surfaces of the cellulose acetate particles, such as, a hydroxyl group, chemically reacting the functional group with a lipophilicity-imparting agent having a functional group reactive with the functional group on the surfaces of the cellulose acetate particles, and thus forming a chemical covalent bond to deposit the lipophilicity-imparting agent on the surfaces of the cellulose acetate particles.

In addition, examples of the technique for the method include a dry treatment method, a wet treatment method, a spray drying method, a gas-phase method, and a mechanochemical method.

The dry treatment method refers to a method of directly mixing the lipophilicity-imparting agent and the cellulose acetate particles. For the cellulose acetate particles present as an aggregate in which the primary particles aggregate, the dry treatment method may fail to sufficiently coat the lipophilicity-imparting agent on the surfaces of the particles.

The wet treatment method refers to a method of diluting the lipophilicity-imparting agent with a suitable solvent or dispersion medium, mixing the diluted liquid with the cellulose acetate particles, and then evaporating to remove the solvent or dispersion medium. After the evaporation and removal, the lipophilicity-imparting agent may be baked to the cellulose acetate particles by heat treatment. The solvent may be an organic solvent, such as ethanol, isopropyl alcohol, n-hexane, benzene, and toluene. The wet treatment method is preferred in terms of being able to more uniformly deposit and coat the lipophilicity-imparting agent on the particle surfaces than the dry treatment method.

The spray drying method refers to a method of mixing the lipophilicity-imparting agent and a solvent or dispersion medium to prepare a slurry, spraying this slurry onto the cellulose acetate particles, and drying in a short time to remove the solvent or dispersion medium. The slurry may be appropriately adjusted using a solvent or dispersion medium to a low viscosity so that the slurry can be sprayed. The spray drying method is also preferable because the method is capable of uniformly depositing and coating the lipophilicity-imparting agent on the particle surfaces. Here, when the slurry is spray-dried all at once by the spray drying method, the particles aggregate, but this aggregate is broken by a shear force.

In addition, depending on the type or concentration of the lipophilicity-imparting agent, the lipophilicity-imparting agent may act as a binder and cause the particles to easily aggregate, leading to an unsmooth touch as if touching hard particles. In such a case, the wet treatment method is less suitable, and the spray drying method is preferred. Furthermore, the spray drying method is a very effective means when coating a microcrystal that becomes colloidal in water or a compound that dissolves in water on the surfaces of the cellulose acetate particles.

The spray drying method is also preferably used for the cellulose acetate particles containing cellulose acetate with low degree of substitution and thus soluble in water.

Examples of the lipophilicity-imparting agent include a lipid component, a silicone-based component, a metal soap-based component, a fluorine-based component, an amino acid-based component, a lecithin-based component, and a ceramide-based component. One lipophilicity-imparting agent may be used alone, or two or more in combination.

Among them, the lipophilicity-imparting agent preferably contains a silicone-based component. This is because the silicone-based component is physiologically inert and relatively stable in various solvents and at various temperatures, thus allowing a wet surface treatment using various conditions, solvents, and the like to be easily performed, and the silicone-based component easily exhibit the function even with a small amount of addition.

Use of a lipid component as the lipophilicity-imparting agent is described in detail. The melting point of the lipid component is preferably not lower than 50°C and not higher than 80°C, and more preferably not lower than 60°C and not higher than 70°C.

When a fat is used as the lipid component, the melting point may be from 50 to 70°C, the solid fat content at 35°C may be from 50 to 100, the peroxide value may be not greater than 0.5, and the iodine value is not greater than 0.8. The fat having a melting point lower than 50°C may readily solidify the resulting particles containing cellulose acetate in storing the particles at ordinary temperature. The fat having a melting point higher than 70°C would lead to difficulty in uniformly coating the cellulose acetate particles. In addition, the fat having a solid fat content at 35°C of less than 50 would lead to difficulty in uniformly coating the cellulose acetate particles. Furthermore, the fat having a peroxide value greater than 0.5 would accelerate the oxidation of the resulting particles containing cellulose acetate in storing the particles at ordinary temperature.

Use of a silicone-based component as the lipophilicity-imparting agent is described in detail. The kinematic viscosity of the silicone-based component may be from 1 to 1000 mm²/s (cSt) (25°C) or from 20 to 200 mm²/s (cSt) (25°C).

The kinematic viscosity of the silicone-based component material can be measured with a rheometer.

The boiling point of the silicone-based component may be not lower than 150°C, not lower than 180°C, or not lower than 200°C, and may be not higher than 300°C.

For example, the surface treatment can be performed by adding an appropriate amount of a silicone-based component to the cellulose acetate particles, mixing, and then heating the resulting particles. In particular, when methicone, dimethicone, and hydrogen dimethicone (a (dimethicone/methicone) copolymer) are used as the silicone-based component, the heating conditions may be a temperature of 105°C for 18 hours.

When dimethiconol is used as the silicone-based component, the dimethiconol may be a silicone with a molecular weight from 300 to 200000 having a hydroxyl group at both ends of the linear dimethylpolysiloxane backbone. Dimethiconol with a molecular weight of less than 300 may have high volatility, and increase the loss in the surface treatment and leading to difficulty in obtaining a product with a certain quality. Dimethiconol with a molecular weight of greater than 200000 would have high viscosity, poor reactivity, and thus difficulty in application in terms of handling.

Examples of the dimethiconol include dimethiconol in the form of oil, a dilution with another silicone oil, or a dimethiconol emulsion composed of a emulsion composition with water. Examples of the emulsion of dimethiconol include an emulsion obtained by mechanically emulsifying oil of dimethiconol and an emulsion obtained by emulsion polymerization using a low molecular weight siloxane as a starting material. Any type of emulsion may be used as long as the emulsifier used for emulsification is highly safe. Dimethiconol in the form of oil or emulsion can be suitably used.

Use of a metal soap-based component as the lipophilicity-imparting agent is described in detail. Metal soaps are metal salts of long chain hydrocarbon carboxylic acids as well known. This carboxylic acid group is adsorbed to a hydroxyl group remaining on the surfaces of the cellulose acetate particles and is fixed on the particle surfaces. Using a metal soap-based component as the lipophilicity-imparting agent allows the long-chain hydrocarbon group to be present on the surfaces of the particles, thus improving the dispersibility in an oily component. Furthermore, using the metal soap-based component also reduced an absorption amount of an oily component of the cellulose acetate particles. The absorption amount of an oily component is one of the very important factors especially for powder cosmetics. The particles with a large absorption amount of an oily component may cause a phenomenon (caking phenomenon) in which the surface of the powder cosmetic pressed in a cake-like form becomes hard, and an amount required cannot be collected at the time of use, leading to difficulty in using. In addition, also a liquid cosmetic would not be in a uniform state or would have too high viscosity even with an increased proportion of an oily component.

Here, surface treatment using isopropyl titanium triisostearate or the like among metal soap-based components may be referred to as alkyl titanate treatment.

Use of a fluorine-based component as the lipophilicity-imparting agent is described in detail. Use of a perfluoroalkyl phosphate ester as the fluorine-based component can simultaneously impart water repellency and oil repellency to the particles containing cellulose acetate. A phosphate ester group is a functional group that can be stably adsorbed to acid, and thus the fluorine-based component hardly peels off from the cellulose acetate particles due to physical factors, such as shear, and chemical factors, such as a solvent and heat.

Examples of a method of specific treatment for surface-treating the cellulose acetate particles using a fluorine-based component include the following method. Examples include a method of adding water to the cellulose acetate particles to form a slurry state, gradually poring an aqueous solution of a fluorine-based component to the slurry and mixing them, then acidifying the mixture, and allowing the mixture to stand at ordinary temperature or high temperature.

This is a method of dispersing, for example, 95 parts by weight of the cellulose acetate particles and 5 parts by weight of a fluorine-based component (which can be exemplified by Asahi Guard AG530 (available from Asahi Glass Co., Ltd.)) in water, then acidifying and heating the system.

Surface treatment using a perfluoroalkyl phosphate ester can be performed by subjecting water colloid of a perfluoroalkyl phosphate ester diethanolamine salt to an acidic condition in the presence of the cellulose acetate particles to disintegrate this water colloid and having the perfluoroalkyl phosphate moiety to be adsorbed on the particle surfaces.

The formulation containing the particles containing cellulose acetate having water repellency and oil repellency provides a cosmetic composition, such as a foundation, preventing makeup smearing on the skin. The perfluoroalkyl phosphate ester has oil repellency but can be dispersed in an oily component, such as perfluoropolyether.

Use of an amino acid-based component as the lipophilicity-imparting agent is described in detail. A dry blending method can be used. Specifically, examples include a method of thoroughly dispersing the cellulose acetate particles in a dilute aqueous solution of a potassium salt of N-cocoyl glutamic acid, then mixing the dispersion liquid and a solution of Nε-lauroyl-L-lysine dissolved in a sodium hydroxide solution of pH 13, adding a 1 N hydrochloric acid dropwise under stirring to neutralize the mixture, and thereby polymerizing the amino acid to be deposited on the surfaces of the cellulose acetate particles.

Use of a lecithin-based component as the lipophilicity-imparting agent is described in detail. In particular, examples include using hydrogenated lecithin as the lecithin-based component. The cellulose acetate particles are suspended in water at about from 3 to 30 wt.%, and hydrogenated lecithin corresponding to 1.0 to 30 wt.% relative to the cellulose acetate particles is added and thoroughly stirred to make the mixture uniform. Furthermore, a 1 to 30 wt.% aqueous solution of a soluble salt of aluminum, magnesium, calcium, zinc, zirconium, titanium and the like, is added dropwise over time to be from 0.1 to 2 equivalents relative to hydrogenated lecithin over time. This turns hydrogenated lecithin into a water-insoluble metal salt and hydrogenated lecithin is completely adsorbed on the cellulose acetate particle surfaces. Cellulose acetate particles treated with hydrogenated lecithin can be obtained by filtering with a Nutsche or the like and drying the resulting cake at 80 to 100°C.

Use of a ceramide-based component as the lipophilicity-imparting agent is described in detail. Preferred is a method of compositing a ceramide-based component and the cellulose acetate particles by a mechanochemical method (dry method), particularly by a high-speed airflow impact method. This high-speed airflow impact method is a method of treating a ceramide-based component and cellulose acetate particles using a hybridizer. In the hybridizer, the actions of the rotor rotating at high speed and a circulation circuit efficiently transmit mechanical thermal energy mainly due to the impact force to the individual particles to be composited, thus forming composite particles in which the cellulose acetate particles are coated with the ceramide-based component.

In addition, another method can be employed, the method of emulsifying an oil phase containing a ceramide, a lauroylsarcosine ester, and a hydrogenated phospholipid and an aqueous phase to prepare an emulsion composition, mixing the emulsion composition and the cellulose acetate particles, and then drying and removing water. This method treats the particle surfaces more uniformly.

In addition to a ceramide, a lauroylsarcosine ester, and a hydrogenated phospholipids, the oil phase can contain, for example, an oil agent, such as hydrocarbons, fats, waxes, hydrogenated oils, ester oils, fatty acids, higher alcohols, silicone oils, fluorine-based oils, and lanolin derivatives, as an oil agent typically used in cosmetics, regardless of the origin, such as animal oils, vegetable oils, and synthetic oils, as well as properties, such as solid oils, semi-solid oils, liquid oils, and volatile oils.

The oil agent is specifically exemplified by hydrocarbons, such as liquid paraffin, squalane, petrolatum, polyisobutylene, polybutene, paraffin wax, ceresin wax, microcrystalline wax, and Fischer-Tropsch wax; fats, such as Japanese wax, olive oil, castor oil, mink oil, and macadamia nut oil; waxes, such as montan wax, beeswax, carnauba wax, candelilla wax, and spermaceti wax; esters, such as cetyl isooctanoate, isopropyl myristate, isopropyl palmitate, octyldodecyl myristate, glyceryl trioctanoate, diglyceryl diisostearate, diglyceryl triisostearate, glyceryl tribehenate, pentaerythritol rosinate ester, neopentyl glycol dioctanoate, cholesterol fatty acid ester, and di(cholesteryl-behenyl-octyldodecyl) N-lauroyl-L-glutamate; fatty acids, such as stearic acid, lauric acid, myristic acid, behenic acid, oleic acid, rosin acid, and 12-hydroxystearic acid; higher alcohols, such as stearyl alcohol, cetyl alcohol, lauryl alcohol, oleyl alcohol, isostearyl alcohol, behenyl alcohol, and hohoba alcohol; silicones, such as low-polymerized dimethylpolysiloxane, highly-polymerized dimethylpolysiloxane, methylphenylpolysiloxane, decamethylcyclopentasiloxane, octamethylcyclotetrasiloxane, and fluorine-modified silicone; fluorine-based oil agents, such as perfluoropolyether, perfluorodecane, and perfluorooctane; lanolin derivatives, such as lanolin, lanolin acetate, isopropyl lanolin fatty acid, and lanolin alcohol. The oil phase may contain one of these oil agents alone or two or more in combination.

On the other hand, the aqueous phase contains water as an essential component and may additionally contain an aqueous component; such as polyhydric alcohols, such as propylene glycol, dipropylene glycol, and 1,3-butylene glycol; glycerins, such as glycerin and polyglycerin; and lower alcohols, such as ethanol.

The content of water in the emulsion composition is preferably from 70 to 95 wt.% and more preferably from 80 to 90 wt.%. The emulsion composition containing water in such a range is in a good emulsified state.

### Production of cellulose acetate particles

A method for producing cellulose acetate particles may include: mixing cellulose acetate having a total degree of acetyl substitution of not less than 0.7 and not greater than 2.9 and a plasticizer to prepare cellulose acetate impregnated with the plasticizer; kneading the cellulose acetate impregnated with the plasticizer and a water-soluble polymer at not lower than 200°C and not higher than 280°C to prepare a dispersion containing the cellulose acetate impregnated with the plasticizer as a dispersoid; and removing the water-soluble polymer from the dispersion.

### Preparation of cellulose acetate impregnated with plasticizer

In preparation of the cellulose acetate impregnated with the plasticizer, cellulose acetate having a total degree of acetyl substitution of not less than 0.7 and not greater than 2.9 and a plasticizer are mixed.

The cellulose acetate having a total degree of acetyl substitution of not less than 0.7 and not greater than 2.9 can be produced by a well-known method for producing cellulose acetate. Examples of such a production method include what is called an acetic acid method in which acetic anhydride is used as an acetylating agent, acetic acid as a diluent, and sulfuric acid as a catalyst. The basic processes of the acetic acid method include: (1) activation including disintegrating/grinding a pulp raw material (soluble pulp) having a relatively high α-cellulose content and then spraying and mixing acetic acid; (2) acetylation including reacting the activated pulp from (1) with a mixed acid containing acetic anhydride, acetic acid, and an acetylation catalyst (e.g., sulfuric acid); (3) aging including hydrolyzing cellulose acetate to form cellulose acetate having a desired degree of acetylation; and (4) post-treatment including precipitating the cellulose acetate to separate from the reaction solution after completion of the hydrolysis reaction, then purifying, stabilizing, and drying the cellulose acetate.

The total degree of acetyl substitution of the cellulose acetate is not less than 0.7 and not greater than 2.9, preferably not less than 0.7 and less than 2.6, more preferably not less than 1.0 and less than 2.6, even more preferably not less than 1.4 and less than 2.6, and most preferably not less than 2.0 and less than 2.6. The total degree of acetyl substitution can be adjusted by adjusting the conditions of aging (conditions, such as time and temperature).

Any plasticizer having a plasticizing effect in melt-extruding cellulose acetate can be used without particular limitation. Specifically, the plasticizer exemplified as a plasticizer contained in the cellulose acetate particles can be used alone or two or more in combination.

Among the plasticizers exemplified above, the plasticizer is preferably at least one or more selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; adipate-based plasticizers, such as diisononyl adipate; and phthalate-based plasticizers, such as ethyl phthalate and methyl phthalate; more preferably at least one or more selected from the group consisting of triethyl citrate, acetyl triethyl citrate, acetyl tributyl citrate, triacetin, and diisononyl adipate; and even more preferably at least one or more selected from the group consisting of acetyl triethyl citrate, triacetin, diacetin, and diethyl phthalate. Note that a phthalate-based plasticizer must be used with care because of concerns about similarity to environmental hormones.

The plasticizer may be blended in an amount of greater than 0 parts by weight and not greater than 40 parts by weight, not less than 2 parts by weight and not greater than 40 parts by weight, not less than 10 parts by weight and not greater than 30 parts by weight, or not less than 15 parts by weight and not greater than 20 parts by weight relative to 100 parts by weight of the total amount of the cellulose acetate and the plasticizer. Too small blended amount of the plasticizer would tend to reduce the sphericity of the resulting cellulose acetate particles, and too large blended amount of the plasticizer would fail to maintain the shape of the particles, tending to reduce the sphericity.

The cellulose acetate and the plasticizer can be dry-mixed or wet-mixed using a mixer, such as a Henschel mixer. In using a mixer, such as a Henschel mixer, the temperature in the mixer may be a temperature at which the cellulose acetate does not melt, for example, in a range of not lower than 20°C and lower than 200°C.

In addition, the cellulose acetate and the plasticizer may be mixed by melt-kneading. Furthermore, the melt-kneading may be performed in combination with mixing using a mixer, such as a Henschel mixer, and in this case, the melt-kneading is preferably performed after mixing in temperature conditions in a range of not lower than 20°C and lower than 200°C using a mixer, such as a Henschel mixer. The plasticizer and the cellulose acetate become more uniform and mixed well in a short period of time, thus increasing the sphericity of the particles containing cellulose acetate that are finally prepared and improving the tactile sensation and touch feeling of the particles.

The melt-kneading is preferably performed by heating and mixing with an extruder. The kneading temperature (cylinder temperature) of the extruder may be in a range of 200°C to 230°C. The melt-kneading performed even at temperatures in this range can plasticize the cellulose acetate and provide a uniform kneaded product. The melt-kneading performed at too low temperatures may reduce the sphericity of the resulting particles, thus reducing the tactile sensation and the touch feeling. The melt-kneading performed at too high temperatures may cause deterioration or coloration of the kneaded product due to heat and may reduce the viscosity of the melted material, thus failing to sufficiently knead the resin in a twin-screw extruder.

The melting point of the cellulose acetate depends on the degree of substitution but is approximately from 230°C to 280°C and is close to the decomposition temperature of the cellulose acetate. Thus, melt-kneading is typically difficult in this temperature range, but because the cellulose acetate (flakes) impregnated with the plasticizer can reduce the plasticizing temperature. The kneading temperature (cylinder temperature) may be, for example, 200°C in using a twin-screw extruder. The kneaded product may be extruded in a strand shape and formed into a pellet form by hot cutting or the like. The die temperature in this case may be approximately 220°C.

### Preparation of dispersion

In preparing the dispersion, the cellulose acetate impregnated with the plasticizer and a water-soluble polymer are kneaded at not lower than 200°C and not higher than 280°C.

The kneading of the cellulose acetate impregnated with the plasticizer and a water-soluble polymer can be performed with an extruder, such as a twin-screw extruder. The temperature of the kneading refers to the cylinder temperature.

The dispersion may be extruded in a string shape from a die attached to the tip of an extruder, such as a twin-screw extruder, and then cut into pellets. At this time, the die temperature may be not lower than 220°C and not higher than 300°C.

The water-soluble polymer may be blended in an amount of not less than 55 parts by weight and not greater than 99 parts by weight relative to 100 parts by weight of the total amount of the cellulose acetate impregnated with the plasticizer and the water-soluble polymer. The amount is preferably not less than 60 parts by weight and not greater than 90 parts by weight, and even more preferably not less than 65 parts by weight and not greater than 85 parts by weight.

The water-soluble polymer in the present specification refers to a polymer having an insoluble content of less than 50 wt.% when 1 g of the polymer is dissolved in 100 g of water at 25°C. Examples of the water-soluble polymer may include polyvinyl alcohol, polyethylene glycol, sodium polyacrylate, polyvinylpyrrolidone, polypropylene oxide, polyglycerin, polyethylene oxide, vinyl acetate, modified starch, thermoplastic starch, methyl cellulose, ethyl cellulose, hydroxyethyl cellulose, and hydroxypropyl cellulose. Among them, polyvinyl alcohol, polyethylene glycol, and thermoplastic starch are preferred, and polyvinyl alcohol and thermoplastic starch are particularly preferred. Further, the thermoplastic starch can be obtained by a well-known method. For example, reference can be made to JP 06-06307 B, WO 92/04408, etc., and more specifically, for example, a thermoplastic starch prepared by mixing approximately 20% of glycerin as a plasticizer to tapioca starch and kneading them with a twin-screw extruder can be used.

The resulting dispersion is a dispersion containing the water-soluble polymer as a dispersion medium and the cellulose acetate impregnated with the plasticizer as a dispersoid. In other words, the dispersion may be a constitution containing the water-soluble polymer as a sea component and the cellulose acetate impregnated with the plasticizer as an island component. In the dispersion, the kneaded product constituting the island component contains the cellulose acetate and the plasticizer and is mainly spherical.

### Removal of water-soluble polymer

The removal of the water-soluble polymer from the dispersion is described.

The method for removing the water-soluble polymer is not particularly limited as long as the method can dissolve and remove the water-soluble polymer from the particles, but examples include a method of dissolving and removing the water-soluble polymer of the dispersion using a solvent, such as water; an alcohol, such as methanol, ethanol, or isopropanol; or their mixture. Specifically, examples include a method of removing the water-soluble polymer from the dispersion, such as by mixing the dispersion and the solvent and filtering the mixture to take out the filtrate.

In removing the water-soluble polymer from the dispersion, the plasticizer may or may not be removed from the dispersion together with the water-soluble polymer. Thus, the resulting cellulose acetate particles may or may not contain a plasticizer.

The mixing ratio of the dispersion and the solvent is preferably not less than 0.01 wt.% and not greater than 20 wt.%, more preferably not less than 2 wt.% and not greater than 15 wt.%, and even more preferably not less than 4 wt.% and not greater than 13 wt.% relative to the total weight of the dispersion and the solvent. With the mixing ratio of the dispersion of higher than 20 wt.%, the water-soluble polymer may not be sufficiently dissolved and may not be removed by washing, or it may be difficult to separate the cellulose acetate particles not dissolved in the solvent and the water-soluble polymer dissolved in the solvent by an operation, such as filtration or centrifugation.

The mixing temperature of the dispersion and the solvent is preferably not lower than 0°C and not higher than 200°C, more preferably not lower than 20°C and not higher than 110°C, and even more preferably not lower than 40°C and not higher than 80°C. At temperatures lower than 0°C, the water-soluble polymer may not be sufficiently dissolved and may be difficult to remove by washing, and at temperatures higher than 200°C, deformation, aggregation, or the like of the particles may occur, and it may be difficult to take out the particles while maintaining the desired shape of the particles.

The mixing time of the dispersion and the solvent is not particularly limited and is to be appropriately adjusted but may be, for example, for not shorter than 0.5 hours, for not shorter than 1 hour, for not shorter than 3 hours, or for not shorter than 5 hours, and for not shorter than 6 hours.

In addition, the method of mixing is not limited as long as the method can dissolve the water-soluble polymer, but, for example, use of a stirring device, such as an ultrasonic homogenizer or a Three-One Motor, can efficiently remove the water-soluble polymer from the dispersion even at room temperature.

For example, when a Three-One Motor is used as the stirring device, the rotation speed during mixing the dispersion and the solvent may be, for example, not less than 5 rpm and not greater than 3000 rpm. This can more efficiently remove the water-soluble polymer from the dispersion. In addition, this also efficiently removes the plasticizer from the dispersion.

### Examples

Hereinafter, the present invention will be specifically described with reference to examples, but the technical scope of the present invention is not limited by these examples.

### Example 1

### Production of cellulose acetate particles

First, 100 parts by weight of cellulose diacetate (available from Daicel Corporation: total degree of acetyl substitution DS = 2.4) and 25 parts by weight of triacetin as a plasticizer were blended in a dry state, dried at 80°C for not shorter than 12 hours, further stirred and mixed using a Henschel mixer, and a mixture of the cellulose acetate and the plasticizer was obtained. The resulting mixture was fed to a twin-screw extruder (PCM30 available from Ikegai Corp., a cylinder temperature of 200°C, a die temperature of 220°C), melt-kneaded, extruded, pelletized, and a kneaded product was formed.

Then, 32 parts by weight of the pellets of the resulting kneaded product and 68 parts by weight of polyvinyl alcohol (available from The Nippon Synthetic Chemical Industry Co., Ltd., a melting point of 190°C, a saponification degree of 99.1%) as a water-soluble polymer were blended in a dry state, then fed to a twin-screw extruder (PCM30 available from Ikegai Corp., a cylinder temperature of 220°C, a die temperature of 220°C), extruded, and a dispersion was formed.

The resulting dispersion was combined with pure water (a solvent) to give a concentration of not higher than 5 wt.% (weight of dispersion/(weight of dispersion + weight of pure water) x 100), and the mixture was stirred using a Three-One Motor (BL-3000 available from Shinto Scientific Co., Ltd.) at a rotation speed of 500 rpm, at a temperature of 80°C for 3 hours. The solution after stirring was filtered off with filter paper (No. 5A available from ADVANTEC), and the filtrate was taken out. The resulting filtrate was prepared using pure water again to give a concentration of the dispersion of not higher than 5 wt.%, the mixture was further stirred at a rotation speed of 500 rpm, at a temperature of 80°C for 3 hours, and the solution was filtered off to take out the filtrate. This operation was repeated three or more times, and cellulose acetate particles were obtained.

### Surface treatment of cellulose acetate particles

In a 5000-mL separable flask, 900 g of n-hexane (Mw: 86.2) and 12 g of KF-9901 (hydrogen dimethicone: available from Shin-Etsu Chemical Co., Ltd.) were charged and stirred at room temperature to dissolve. Furthermore, 600 g of the resulting cellulose acetate particles were added, stirring was continued at room temperature for 30 minutes to disperse the cellulose acetate particles, and slurry was formed. This slurry was distilled in a water bath at 85°C under normal pressure to remove n-hexane. This turned the entire system into particulate. The particles were then stirred in an oil bath at 120°C for not shorter than 2 hours, dimethicone was baked to the surfaces of the cellulose acetate particles, and surface-treated cellulose acetate particles were obtained.

The surface-treated cellulose acetate particles were measured and evaluated for average particle size, coefficient of variation of particle size, sphericity, degree of surface smoothness, bulk density, plasticizer content, biodegradability, tactile sensation, floatability in water, floatability in isododecane, and contact angle. In addition, the cellulose acetate particles were also measured and evaluated for biodegradability and tactile sensation. The results are shown in Table 1. Each physical property was measured and evaluated by the methods described below.

### Average particle size and coefficient of variation of particle size

The average particle size was measured using dynamic light scattering. First, the sample was adjusted to a concentration of approximately 100 ppm using pure water, and a pure water suspension was prepared using an ultrasonic vibrating device. Then, the particle size volume distribution was determined by laser diffraction ("Laser Diffraction/Scattering Particle Size Distribution Measuring Apparatus LA-960" available from Horiba Ltd., ultrasonic treatment for 15 minutes, a refractive index (1.500, medium (water; 1.333)), and the average particle size was measured. The average particle size (in nm, µm, etc.) herein was the value of the particle size corresponding to 50% of the integrated scattering intensity in the particle size volume distribution. In addition, the coefficient of variation (%) of the particle size was calculated by an equation: standard deviation of particle size/average particle size x 100.

### Sphericity

Using an image of particles observed with a scanning electron microscope (SEM), the major axis length and the minor axis length of 30 randomly selected particles were measured to determine the (minor axis length)/(major axis length) ratio of each particle, and the average value of the (minor axis length)/(major axis length) ratios was taken as the sphericity.

### Degree of surface smoothness

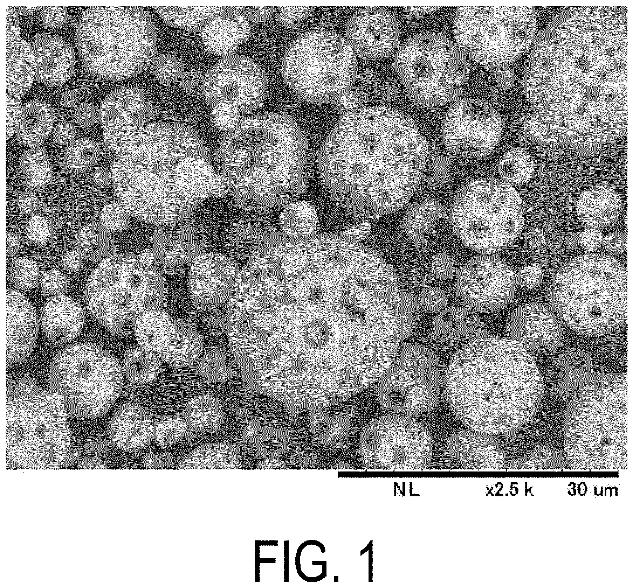

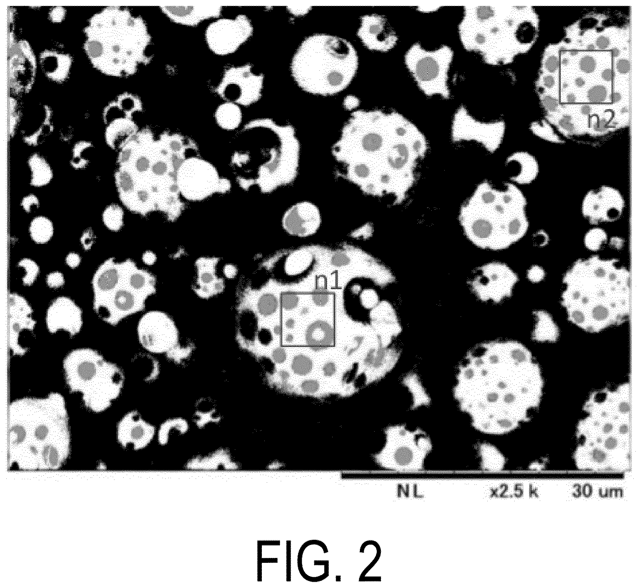

A scanning electron micrograph of the particles was taken at a magnification of 2500 to 5000 x (see FIG. 1 for an example of a micrograph of the cellulose acetate particles), and the image was binarized using an image processing device WinROOF (available from Mitani Corporation) (See FIG. 2 for the binarized image of the micrograph of FIG. 1). They may be any areas each smaller than the particle including the center and/or the vicinity of the center of one particle (e.g., the areas indicated by n1 and n2 in FIG. 2). In addition, the size of the area may be 5 µm square for the particle with a diameter of 15 µm. The area ratio of the portion corresponding to recesses (the shaded portions) of recesses and protrusions in the area was calculated, and the degree of surface smoothness (%) of the one particle was calculated by the following equation.
Degree of surface smoothness of one particle (%) = (1 - area ratio of recesses) x 100 Area ratio of recesses = area of recessed portions in the any area/the any area

The average value of the degree of surface smoothness of randomly selected 10 particle samples, that is, from n1 to 10, was taken as the degree of surface smoothness (%). The higher this numerical value, the higher the degree of surface smoothness is.

### Bulk density

The bulk density was measured according to "JIS K 1201-1".

### Plasticizer content

The plasticizer content (wt.%) was measured by ¹H-NMR measurement.

### Biodegradability

Biodegradability was evaluated by biodegradation rate. The biodegradation rate was measured by a method using activated sludge in accordance with JIS K6950. The activated sludge was obtained from a municipal sewage-treatment plant. About 300 mL of a supernatant (activated sludge concentration: about 360 ppm) obtained by allowing the activated sludge to stand for approximately 1 hour was used per culture bottle. The measurement was started when 30 mg of the sample was stirred in the supernatant, and then the sample was measured every 24 hours until after 720 hours, that is until after 30 days, a total of 31 times. Details of the measurement are as follows. The biochemical oxygen demand (BOD) in each culture bottle was measured using a Coulometer OM3001 available from Ohkura Electric Co., Ltd. The percentage of the biochemical oxygen demand (BOD) to the theoretical biochemical oxygen demand (BOD) in complete degradation based on the chemical composition of each sample was taken as the biodegradation rate (wt.%), and the biodegradability was evaluated as follows.

Excellent: greater than 60 wt.%. Good: not less than 40 wt.% and not greater than 60 wt.%. Marginal: not less than 10 wt.% and less than 40 wt.%. Poor: less than 10 wt.%.

### Tactile sensation

Sensory evaluation was performed according to a panel test by 20 panelists for the tactile sensation of the particles. Panelists were instructed to touch the particles to evaluate comprehensively both smoothness and moist feeling, on a scale with a maximum score of 5 points according to the following criteria, and an average score from 20 panelists was calculated.

Good: 5. Slightly good: 4. Average: 3. Slightly poor: 2. Poor: 1.

### Floatability in water

First, 1 g of the particles and 50 mL of water were mixed and stirred in conditions of a rotation speed of not lower than 100 rpm and a time of not shorter than 30 seconds, and then the mixture was allowed to stand for not shorter than 30 seconds. Particles floating on water were collected, dried, and then the weight was measured. The relative value of the weight of the particles after drying, the particles that floated on water, to the weight of the particles before mixed and stirred with water, the weight defined as 100, was taken as the floatability in water.

### Floatability in isododecane

First, 1 g of the particles and 50 mL of isododecane were mixed and stirred in conditions of a rotation speed of not lower than 100 rpm and a time of not shorter than 30 seconds, and then the mixture was allowed to stand for not shorter than 30 seconds. Particles floating on isododecane were collected, dried, and then the weight was measured. The relative value of the weight of the particles after drying, the particles that floated on isododecane, to the weight of the particles before mixed and stirred with isododecane, the weight defined as 100, was taken as the floatability in isododecane.

### Surface contact angle with water (θ/2 method)

Double-sided tape was applied on a slide glass, 2 g of the particles was uniformly coated on the tape to form a plane. A water droplet was dropped on the plane, and the contact angle of the water droplet was determined by the θ/2 method. The apparatus used to drop the water droplet and measure the contact angle was a fully automatic contact angle meter (analysis software: interFAce Measurement and Analysis System FAMAS): available from Kyowa Interface Science Co., Ltd.).

### Example 2

### Production of cellulose acetate particles

A kneaded product was obtained in the same manner as in Example 1 except for changing triacetin to 22 parts by weight; a dispersion was formed in the same manner as in Example 1 except for changing the pellets of the resulting kneaded product to 34 parts by weight and polyvinyl alcohol to 66 parts by weight; and the resulting dispersion was combined with pure water to give a concentration of not higher than 5 wt.%, and cellulose acetate particles were obtained in the same manner as in Example 1 except for stirring the mixture at a rotation speed of 200 rpm at a temperature of 80°C for 5 hours.

### Surface treatment of cellulose acetate particles

The resulting cellulose acetate particles were treated in the same manner as in Example 1, and surface-treated particles were obtained.

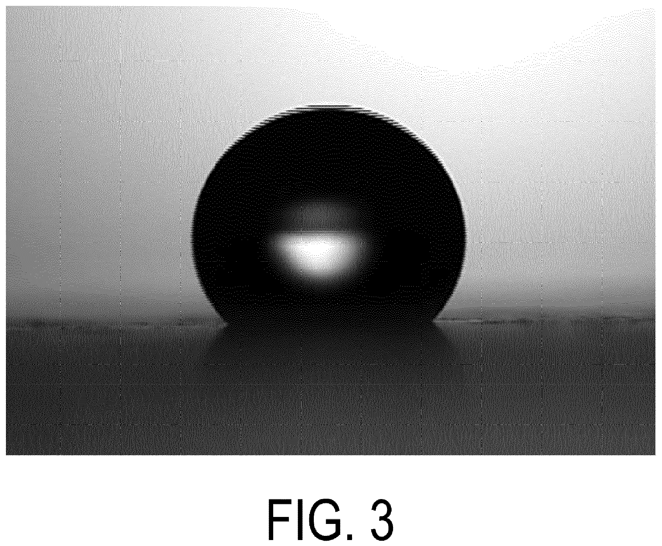

The resulting cellulose acetate particles and the surface-treated cellulose acetate particles were measured and evaluated for each physical property by the above methods each in the same manner as in Example 1. The results are shown in Table 1 and illustrated in FIG. 3.

### Example 3

### Production of cellulose acetate particles

Cellulose acetate particles were obtained in the same manner as in Example 1 except for mixing 20 parts by weight of glycerin with 80 parts by weight of thermoplastic starch (pregelatinized tapioca starch, available from Sanwa Starch Co., Ltd.) as a water-soluble polymer instead of polyvinyl alcohol to make 100 parts by weight of a mixture and using 68 parts by weight of the mixture to form a dispersion.

### Surface treatment of cellulose acetate particles

The resulting cellulose acetate particles were treated in the same manner as in Example 1, and surface-treated particles were obtained.

The resulting cellulose acetate particles and the surface-treated cellulose acetate particles were measured and evaluated for each physical property by the above methods each in the same manner as in Example 1. The results are shown in Table 1.

### Comparative Examples 1 to 3

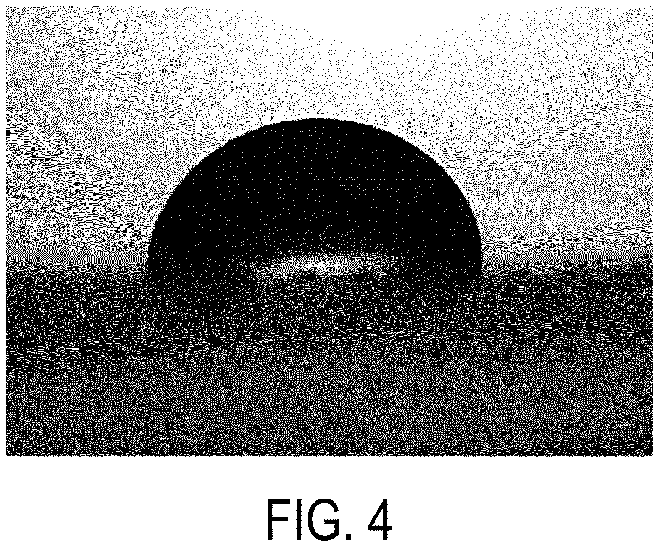

Cellulose acetate particles were obtained each in the same manner as in Examples 1 to 3; however, the cellulose acetate particles were not surface-treated. The resulting cellulose acetate particles were measured and evaluated for each physical property by the above methods. The results are shown in Table 1 and illustrated in FIG. 4.

**[Table 1]**

| | Example 1 | Example 2 | Example 3 | Comparative Example 1 | Comparative Example 2 | Comparative Example 3 |
|---|---|---|---|---|---|---|
| Total degree of acetyl substitution (DS) | 2.4 | 2.4 | 2.4 | 2.4 | 2.4 | 2.4 |
| Plasticizer | Triacetin | Triacetin | Triacetin | Triacetin | Triacetin | Triacetin |
| Average particle size (µm) | 5.2 µm | 7.1 µm | 6.8 µm | 5.2 µm | 7.1 µm | 6.8 µm |
| Coefficient of variation of particle size (%) | 36 | 37 | 39 | 36 | 37 | 39 |
| Sphericity | 0.98 | 0.97 | 0.95 | 0.98 | 0.97 | 0.95 |
| Degree of surface smoothness (%) | 100 | 100 | 100 | 100 | 100 | 100 |
| Bulk density | 0.50 | 0.47 | 0.45 | 0.60 | 0.58 | 0.58 |
| Plasticizer content (wt.%) | <0.01 | <0.02 | <0.01 | <0.01 | <0.02 | <0.01 |
| Biodegradability (before surface treatment) | Excellent | Excellent | Excellent | Excellent | Excellent | Excellent |
| Biodegradability (after surface treatment) | Good | Good | Good | - | - | - |
| Tactile sensation (before surface treatment) | 4.6 | 4.8 | 4.4 | 4.6 | 4.8 | 4.4 |
| Tactile sensation (after surface treatment) | 4.5 | 4.9 | 4.3 | - | - | - |
| Floatability in water | 100 | 100 | 100 | Good | Good | Good |
| Floatability in isododecane | Good | Good | Good | 100 | 100 | 100 |
| Contact angle (θ/2 method) (°) | 130 | 127 | 132 | 80 | 88 | 92 |

As shown in Table 1, the particles of Examples were found to have excellent biodegradability and tactile sensation comparable to those of the Comparative Examples. In addition, as shown in Table 1 and illustrated in FIG. 4, all of the particles of Comparative Examples sank in water, the surface contact angles with water were from 80 to 92°, and all sank in water. In contrast, all of the particles of Examples floated on water, the surface contact angles with water were values sufficiently greater than 100°, and all sank in isododecane. The particles of the Examples are thus found to have excellent lipophilicity.

## Claims

1. Particles containing cellulose acetate, wherein
the particles have an average particle size of not less than 80 nm and not greater than 100 µm, a sphericity of not less than 0.7 and not greater than 1.0, a degree of surface smoothness of not less than 80% and not greater than 100%, and a surface contact angle with water of not less than 100°; and
a total degree of acetyl substitution of the cellulose acetate is not less than 0.7 and not greater than 2.9.

2. The particles according to claim 1, wherein the surface contact angle with water is not less than 120°.

3. The particles according to claim 1 or 2, wherein the total degree of acetyl substitution of the cellulose acetate is not less than 2.0 and less than 2.6.

4. The particles according to any one of claims 1 to 3, wherein
the particles contain a plasticizer, and
a content of the plasticizer is not greater than 1 wt.% relative to a weight of the particles.

5. The particles according to claim 4, wherein the plasticizer is at least one or more selected from the group consisting of a citrate-based plasticizer, a glycerin ester-based plasticizer, an adipate-based plasticizer, and a phthalate-based plasticizer.

6. The particles according to claim 5, wherein the glycerin ester-based plasticizer is triacetin.

7. A cosmetic composition containing the particles described in any one of claims 1 to 6.

8. A method for producing particles, the particles described in claim 1, the method comprising surface-treating cellulose acetate particles with a lipophilicity-imparting agent, wherein
the cellulose acetate particles have an average particle size of not less than 80 nm and not greater than 100 µm, a sphericity of not less than 0.7 and not greater than 1.0, and a degree of surface smoothness of not less than 80% and not greater than 100%; and
a total degree of acetyl substitution of the cellulose acetate is not less than 0.7 and not greater than 2.9.

9. The method for producing particles according to claim 8, wherein the lipophilicity-imparting agent comprises a silicone-based component.

10. The method for producing particles according to claim 9, the particles comprising cellulose acetate, wherein the surface treatment is a surface treatment by a wet treatment method.
